# EUROPEAN PATENT APPLICATION

(11) **EP 3 009 832 A1**
(43) Date of publication of application: **20.04.2016**
(21) Application number: 15190028.9
(22) Date of filing: 18.12.2009
(51) Int. Cl.: G01N 21/65, A61B 5/00

(54) **A CYTOLOGICAL SYSTEM AND METHOD FOR ANALYZING A BIOLOGICAL SAMPLE BY RAMAN SPECTROSCOPY**

(30) Priority: 18.12.2008 GB 0823071
(62) Divisional of application: 09806090.8
(71) Applicant: Dublin Institute of Technology, Dublin 6 (IE)
(72) Inventor: LYNG, Fiona, Dublin, 8 (IE); BYRNE, Hugh, Dublin, 8 (IE); Ó FAOLÁIN, Eoghan, Dublin, 8 (IE); CLARKE, Colin, Dublin, 8 (IE); OSTROWSKA, Kamila, Dublin, 8 (IE)
(74) Representative: Walsh, Marie Goretti

(57) **Abstract**

The present application provides a system for analyzing a PAP smear sample or a biopsy sample on a slide. The system suitably comprises a controller, a stage for receiving the slide, a microscope for viewing the slide and a Raman spectroscopy device. The Raman spectroscopy device shares the central optical axis of the microscope. The controller is adapted to cause the stage to move an identified area of interest on the slide to be in line with the central optical axis and to cause a spectrum to be obtained by the Raman spectroscopy device for the area of interest. An analysis module is then used to determine whether the spectrum falls within one or more predefined classes of cell. The application also describes a method which consolidates the sample in a centrifuge to pellet form. The pellet is then analysed using Raman spectroscopy, without the requirement for microscopic analysis and extendable to a fibre optic probe modality.

## Description

### Background

Cancer is a class of diseases in which a group of cells display uncontrolled growth and is responsible for approximately 13% of all deaths. As early detection of cancer results in improved survival rates and less drastic treatments, significant research has been conducted in the area of diagnosis.

The most common method of diagnosis involves performing a biopsy. A biopsy involves the removal of cells or tissues for examination, typically by means of a scalpel or a needle. The biopsy is an extremely localised test since the person taking the sample is targeting a specific area. As a result, it is clear what cells are contained within the biopsy. In addition, the tissue has a very defined architecture which is well known to the histopathologist. Once a sample has been taken it undergoes a series of steps including fixation, embedding, sectioning using a microtome and finally staining, before the samples are viewed (typically with an optical microscope). This work is typically carried out by histopathologists. Since a biopsy is in effect a surgical procedure, biopsies are a relatively low throughput system. In addition, the number of different identifiable cell types\structures is relatively low (3 or so).

Existing diagnosis methods are low throughput. As a result, less accurate but higher throughput methods are employed for screening purposes. Thus in the case of cervical cancer, which is the second most deadly cancer in women, the currently preferred screening method is the smear test (Papanicolaou smear). In a smear test, samples are collected from the outer opening of the cervix using a spatula and then visually inspected. The sample is stained as unstained cells may not be visualized with light microscopy. The cells are placed on a glass slide and checked for abnormalities in the laboratory. Automated, computer image analysis systems have been developed for screening although these all operate with human oversight, whereby the automated system performs an initial pass to be followed by a human operator viewing the images in situations where the image analysis indicates a potential abnormality. However, even then the screening process is also labour intensive requiring visual inspection of each slide. Smear tests in contrast to biopsies are intended to be for mass screening. A significant difference between a biopsy and a smear test sample is the significant number of different cells and potential contaminants present in a smear test by virtue of the non-targeted approach to collection of the sample. Obviously, the more complicated the mix, the more complicated the analysis. As a result, current smear tests are prone to sampling / reading errors and false negatives / positives are common. Where a smear test is identified as abnormal, it is followed by colposcopic examination, biopsy and histological confirmation of the clinical diagnosis. This involves the visual examination of histological sections by highly trained personnel. The grading characteristics are quite subjective and pre-malignancy may not be visually perceptible at all. Moreover, the smear test has a false negative error rate of 15-59% resulting in significant unnecessary further investigations.

One area that has been investigated for use in diagnosis is that of spectroscopy, of which a first type is fluorescence spectroscopy. In fluorescence spectroscopy, photons of light are absorbed. By analysing the different frequencies of light emitted in fluorescence spectroscopy, information on the molecular structure being examined may be obtained. An example of fluorescence spectroscopy is disclosed in US5,945,674 which describes a method of detecting cellular types. Another type of spectroscopy is IR spectroscopy which measures responses in the IR region. Another form of spectroscopy is Raman spectroscopy. Raman spectroscopy is a spectroscopic technique (vibrational spectroscopy) which relies on Raman scattering by a sample of monochromatic light from a laser. Raman scattering is not simply a direct alternative to other techniques, such as IR spectroscopy as typically bands that are Raman active are usually weak in IR and vice versa. In Raman scattering, a defined amount of energy is transferred from the photon to the molecule in which a vibrational mode is excited. The exact energy required to excite a molecular vibration depends on the masses of the atoms involved in the vibration and the type of chemical bonds between these atoms and may be influenced by molecular structure, molecular interactions and the chemical microenvironment of the molecule. Therefore, the positions, relative intensities and shapes of the bands in a Raman spectrum carry detailed information about the molecular composition of the sample. This may be beneficially employed for example as disclosed in US2008/192246 where Raman spectroscopy is employed in a complex system for identifying detecting hazardous agents Moreover, it will be appreciated that changes in spectral response from different patient samples may be used to characterise disease. Whilst the results of Raman spectroscopy have been promising in terms of disease diagnostics, the equipment commonly employed is very large in size and also expensive (approx. €250,000). It is also complicated to use, requiring specialised training.

Some research has been developed which combines the use of both fluorescence and Raman spectroscopy techniques. In particular, the University of Texas have published research primarily directed at developing a fibre optic probe which might be inserted internally to examine a particular cell site to investigate whether the cells at that site were cancerous or not (as disclosed in WO9748329, US6258576 and US5697373, for example, which discuss the possibility of employing Raman spectroscopy in combination with fluorescence spectroscopy in which the screening process is primarily carried out using fluorescence spectroscopy to identify normal tissue and low and high grade lesions and Raman spectroscopy to identify inflammation and metaplasia).

The present application seeks to improve the performance of existing smear techniques.

### Summary

The present application seeks to improve the performance of existing smear techniques using low resolution, low cost RAMAN spectroscopy. As it is believed that there would be significant reluctance in the medical community to the complete replacement of existing techniques, a first embodiment provides a system which may be employed to automate the elimination of definite negatives from a set of specimens, thus providing a skilled user with more time for the analysis of possible positives. In particular, this first embodiment provides a diagnostic instrument comprising an optical microscope for viewing a sample comprising cells, an interface for receiving a user input identifying an area of the sample for further investigation, a Raman spectroscopy device to obtain a spectral signature for the identified area, a library of known signatures for predefined classes of cell, a classifier for comparing the obtained signature with the known signatures to classify the cell within one of the predefined classes.

The invention also extends to the following statements.
1. A system for analyzing a PAP smear sample on a slide, the system comprising: a controller, a stage for receiving the slide, a microscope for viewing the slide, the microscope having a central optical axis, a Raman spectroscopy device sharing the central optical axis of the microscope, wherein the controller is adapted to cause the stage to move an identified area of interest on the slide to be in-line with the central optical axis and to cause a spectrum to be obtained by the Raman spectroscopy device for the area of interest, the system further comprising an analysis module for determining whether the spectrum falls within one or more predefined classes of cell.
2. A system according to statement 1, further comprising a graphical user interface comprising a window display the view from the microscope, wherein the interface is configured to allow a user to use a pointer to identify the area of interest.
3. A system according to statement 2, wherein the result of the determination of whether the spectrum falls within one or more predefined classes of cell is displayed within the graphical user interface.
4. A system according to statement 1, wherein the analysis module is configured to perform image analysis on an image acquired by the microscope to identify areas of interest.
5. A system according to statement 4, wherein the image analysis identifies cells as areas of interest.
6. A system according to any preceding statement, wherein the system further comprises a light source for illuminating the slide, wherein the controller is adapted to switch off the light source when operating the Raman spectroscopy device.
7. A system according to any preceding statement further comprising a moveable mirror for switching the optical path between the microscope and the Raman spectroscopy device, wherein the moveable mirror is responsive to the controller.
8. A system according to any preceding statement, wherein the one or more predefined classes comprise one or more of the following:
   a) normal, b) invasive carcinoma and c) cervical intraepithelial neoplasia (CIN).
9. A system according to statement 8, wherein the CIN class is further delineated into the classes of:
   a) CIN I, b) CIN II and c) CIN III.

A system according to any preceding statement wherein the Raman spectroscopy device is a low resolution Raman spectroscopy device.

### Description of Drawings

Figure 1 is an exemplary block diagram of a system according to the present application,
Figure 2 is a first mode of operation of the system of Figure 1,
Figure 3 is a second mode of operation of the system of Figure 1,
Figures 4A illustrates high resolution Raman spectra of normal epithelial tissue (bottom) and invasive carcinoma (top) and Figure 4B illustrates a PCA-LDA plot showing differentiation between normal epithelial tissue (N), invasive carcinoma (T) and CIN tissue (C).
Figures 5A illustrates Raman spectra of normal cervical tissue and Figure 5B illustrates invasive carcinoma (figure 5B) at high resolution (A, C) and low resolution (B, D),
Figure 6A illustrates Raman spectra of a normal cervical smear sample and
Figure 6B illustrates an abnormal (CIN III) smear sample at high resolution (A, C) and low resolution (B, D)
Figure 7A illustrates multivariate analysis of the data showing differentiation between normal epithelial tissue (1), invasive carcinoma (2) and CIN tissue (3) and Figure 7B illustrates normal (negative), negative / reactive changes, inflammation, borderline nuclear abnormalities and CIN III smear samples,
Figure 8 is an exemplary graphical user interface for use with the system of Figure 1,
Figure 9 is a process flow diagram including an exemplary analysis technique for use within the present application, and
Figure 10 is a flow chart of a further embodiment of the present application.

### Detailed Description of Drawings

The present inventors have realised that low resolution instruments may be employed with suitable modification to provide an extremely powerful diagnostic tool for cervical smear or other cytological / biological samples. This realisation has led to the development of (a) low cost, versatile instrumentation, ideally suited for clinical applications. By low resolution, a spectral resolution of worse than 10 wavenumbers is meant.

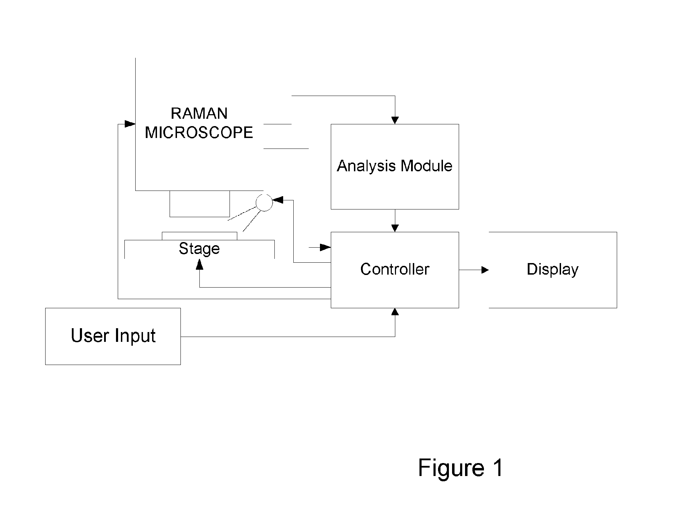

An exemplary system is now described which is practical for a hospital laboratory and is intended to resemble existing microscope based optical diagnostic systems as would be familiar to those skilled in the art. The system, as illustrated in Figure 1, comprises a Raman microscopy system which has been adapted to include a graphical user interface viewable on a display, an analysis module for analysing the spectrum provided by the Raman microscopy system, a controller and a controllable stage. The system allows a user to point and click on an area of interest, record a Raman spectrum or a number of spectra from individual cells.

The Raman microscopy system comprises an optical microscope integrated with a low resolution Raman spectrometer. As would be typical in such systems, the optical microscope and Raman spectrometer desirably, for alignment purposes, have a common objective lens. A moveable mirror or similar feature is provided to switch the optical path between that of the viewing optics of the microscope and those of the Raman spectrometer. The mirror is switched in response to a signal from the controller. The Raman spectrometer is configured such that the area of measurement corresponds to a central region of the viewable area of the microscope. A movable stage is provided below the objective lens and is suitably configured to receive a slide. This may be, for example, by means of a recess in the shape of the slide or guides on the surface. The movable stage is responsive to the controller to effect motion of the stage and thus the area of the slide under the collection optics.

The stage is effectively a device which may be operated to move in an x-y axis, the stage may use stepper motors or similar to ensure the stage moves to a required position as provided by the controller. The controller may operate the stage in response to a user input for example, by means of a joystick or similar device, or in an automatic fashion as will be described in greater detail below.

A switchable light source is provided for illuminating the sample on a slide. The light source may be switchable directly in response to an input from the controller to illuminate the slide or not. Alternatively, a mechanical or electronic shutter responsive to the controller may be employed to switch on\off the illumination of the slide as required by blocking\unblocking the optical path between the slide and the light source.

Suitably, the user may view the sample through either the eyepieces of the optical microscope or within a window on the display screen (where the image is acquired by a digital camera or similar imaging device).

The operation of the system will now be described with reference to two exemplary modes of operation.

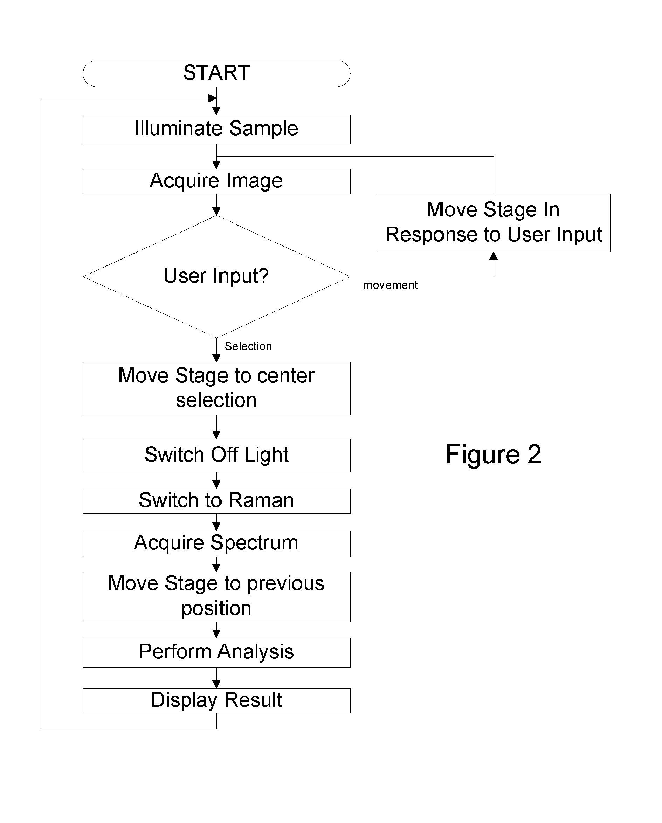

In a first mode of operation, shown in Figure 2, a user places a slide carrying the sample to be investigated on the stage. The optics are switched such the user is able to view the sample under the microscope, for example within a window on the display. Initially, in this mode, the controller operates the stage in response to the user input for example by means of a joystick. This allows a user to view different areas on the slide. As the user views a particular area, they may consider that a particular area within the frame, e.g. a cell is suspicious and worthy of further investigation. The user can position a cursor on the area of interest within the window displaying the microscope view. Once the cursor is positioned, the user can click a mouse button to activate the analysis steps. Once activated, the controller determines the distance both x and y that the stage is required to move the slide to present the area of interest in the center of the optical axis of the Raman microscope. This information is then fed to the stage as a control signal to cause the area of interest to be positioned centrally. The light is then switched off, the optics switched to the Raman spectrometer and the laser activated. The spectrum of the area of interest is then taken. The controller then causes the stage to return to its previous position. The analysis module performs an analysis on the acquired spectrum to compare it with a large library of pre-recorded spectra from a wide sample base including all grades of cervical intraepithelial neoplasia (CIN I, II and III). An algorithm, as will be described below, then classifies the spectrum into the most appropriate group and an identification of the classification result is returned via the graphical user interface. An advantage of this is that a conventionally trained user may rapidly eliminate possible cells using a simple point and click process without any knowledge of spectroscopy.

The system may also be used in a second mode which is a substantially automated mode of operation in which the whole cervical smear may be scanned and abnormal areas identified, highlighted and classified for subsequent review.

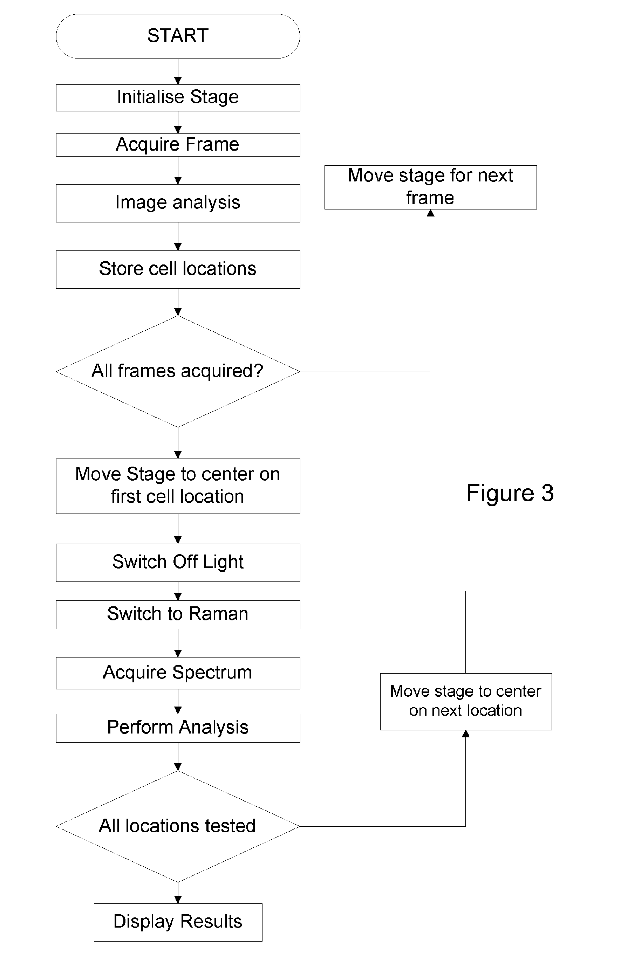

In the second mode of operation, shown in Figure 3, the light source is activated by the controller to illuminate the sample. The entire area of the sample is then captured by the digital camera as a series of frames to provide a digital image of the sample. The frames may be analyzed individually as they are acquired or as a single process on the entire digital image. The digital image may be stored for subsequent viewing by a user. The digital image/individual frames are analyzed using image analysis to identify cells and other features of potential interest within the image. It will be understood that techniques for performing this type of image analysis would be familiar to those skilled in the field and may include, for example, the use of edge and boundary detection techniques. More advanced techniques may also be employed to limit the identification to suspect cells. Where a cell or other feature is identified as being of interest, its position is recorded. This process is repeated for the entire digital image. Once this step is completed, the controller moves the stage to center the first identified location on the optical axis, the light is switched off, the optics switched to the Raman spectrometer and the laser activated. A spectrum of the area of interest is then taken and analyzed as described previously with respect to the first mode and in greater detail below. The result of the analysis is then stored with the location. This process is then repeated for all of the identified locations. Once the measurements and analysis for each location have been completed, the controller may check to determine whether any locations were identified as being cancerous in nature. If not, the system may request the removal of the slide and insertion of the next. More advantageously, an automated feed system may be provided to feed slides in succession. In the event that the system identifies one or more areas as being within a particular cell classification of concern, e.g. cancerous, then a warning or alert may be provided to a user, for example in the form of a message on the display or an audible warning. The interface may then present the user with a sequential view of the identified areas from the digital image to allow the user to confirm the result.

The advantage of using Low Resolution Raman Spectroscopy (LRRS) is that in contrast to previous research, high spectral resolution is not required, thus allowing an instrument to be provided employing low cost lasers and miniature spectrometers thus lowering the size, weight and cost of instrumentation by an order of magnitude.

An in built algorithm for classification of unknown samples compares the spectra to a large database of normal, invasive carcinoma and CIN I, II and III samples and allocates the spectra to the most similar group.
A simple user interface which may be used by a non-specialist allows spectra to be recorded rapidly and easily and the classification may be given as one of five different types: normal / invasive carcinoma / CIN I / CIN II / CIN III. No expertise in spectroscopy is required as spectra are not analysed by the user, but simply fed into the analysis module employing an in built algorithm. This aspect makes the technology ideal for a busy and demanding hospital setting as no new skills would be required and the process would be extremely rapid.

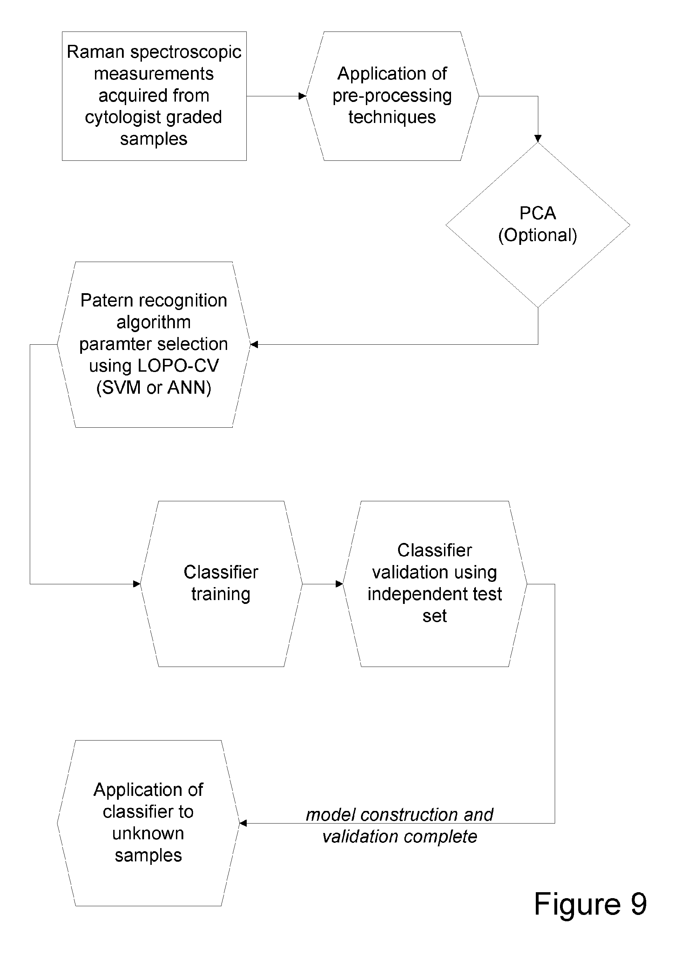

The algorithm techniques described above will now be discussed with reference to Figure 9. Following the acquisition of Raman measurements from a cervical smear sample, a range of data pre-processing techniques (smoothing, normalization, derivatisation) may be applied to the data in order to reduce experimental variance before progressing to multivariate statistical analyses.

Exemplary multivariate statistical techniques that may be employed fall under two main categories, supervised and unsupervised. Unsupervised techniques such as principal component analysis (PCA) assume no prior knowledge of the data under consideration. Mathematical transformation of the spectra allows the data to be described in terms of the co-variations within each spectra permitting closer investigation of the spectral regions resulting in separation between groups. In this application, the use of principal component analysis is primarily applied to reduce the computational intensity required to develop supervised pattern recognition models. An additional benefit of PCA is that noise can be removed from the spectra as the technique reorganises the data with respect to the principal components of variance and therefore the majority of the information contained in the original data is now present in a lower number of vectors, vectors with a low level of variance from the original dataset can be removed with minimal loss of information. A third advantage arising from using PCA is the ability to reveal outliers within a spectral dataset and to remove them, thus increasing the final accuracy of the system. PCA may be used as an initial step in the present application when analyzing data. It may be omitted but this would increase the complexity and time required to conduct the second step considerably.

Once this initial analysis is complete a second multivariate approach is employed using supervised learning algorithms. These techniques would be familiar to persons in the art. Such algorithms may be described as intelligent systems, wherein prior knowledge of the samples is used, specifically cytologist grading of the sample, i.e. the system is initially trained using a series of known results. Hence the patterns within the data may be "learned" and predictive models formed to classify unknown samples. For the current system, a machine learning approach called support vector machines (SVM) may be utilized. In short, the technique locates the optimum linear or nonlinear decision boundary between two or more classes in multidimensional space. The process of developing an SVM model begins with a dataset containing cytologist graded set of cervical smears measured using Raman. PCA may be used to reduce the dimensions of the data, or the entire dimensionality of the data can be used to train the SVM, although SVMs can operate on datasets of large numbers of dimensions without increasing the complexity. The first stage uses leave one patient out cross validation (LOPO-CV) procedure to select the best settings for the SVM. Once the optimum settings have been selected the model is trained. In order to ensure confidence in the model an independent test set validation is carried out. Artificial neural network (ANN) classifiers such as the MLP may also be used for this task. ANNs are loosely based on processes observed in the brain, consisting of highly linked neurons. Each processing unit in the ANN has a weight and bias associated with it in the training phase of the algorithm these weights and biases are modified in order to produce the classification model. The construction and evaluation of an ANN is identical to that of the SVM.

Once the model has been trained, sample results may be applied to the model and a result comprising a classification of the sample obtained.

The medical community is generally considered to be slow to embrace new technology so it is proposed that the first mode of operation would be implemented first with the second mode as users became familiar with the system. Both cervical smears and biopsied material (either fresh tissue sections or dewaxed sections) may be analyzed.

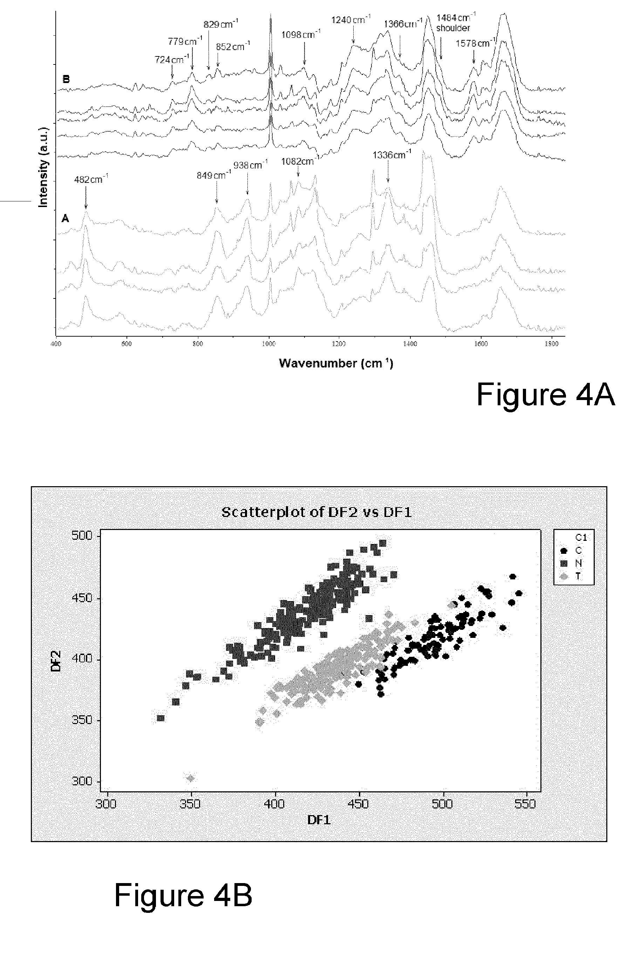

Investigations by the inventors on normal and abnormal cervical tissue sections, using Raman spectroscopy have shown that marked spectroscopic differences between normal and abnormal tissue may be consistently observed and excellent correlation with pathological diagnosis achieved as illustrated in Figure 4.

With leave one out cross validation, a prediction accuracy of 98.8% has been achieved. More importantly, in tests conducted on approximately 300 different invasive carcinoma and CIN (pre-maligant) spectra applied to the discriminant analysis by the inventors, not a single abnormal spectrum was misclassified as normal.

Sensitivity and specificity are the most widely used statistics used to describe a diagnostic test. Sensitivity refers to the probability of a positive test among patients with disease, while specificity refers to the probability of a negative test among patients without disease. Excellent sensitivity and specificity values were obtained (table 1) indicating that Raman spectroscopy coupled with multivariate statistical analysis is an extremely reliable method for cervical cancer diagnosis.

**Table 1 Sensitivity and specificity values for normal epithelial tissue, invasive carcinoma and CIN tissue**

| | **Sensitivity** | **Specificity** |
|---|---|---|
| Normal | 99.5% | 100% |
| Invasive carcinoma | 98.5% | 99% |
| CIN | 99% | 99.2% |

As described above, the inventors have collected convincing evidence that vibrational spectroscopy may be employed within a diagnostic tool for early detection of cancer. Raman spectroscopy is a non-destructive and non-invasive technique making it ideal for a hospital setting. This optical technique is also easily adaptable to endoscopes, making it a versatile tool for a variety of cancers.

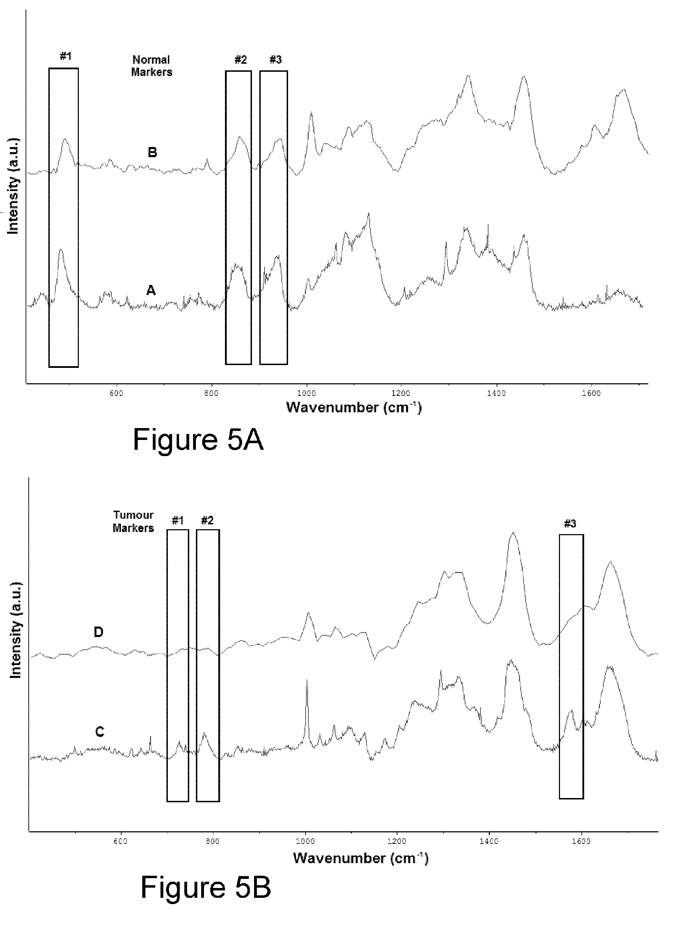

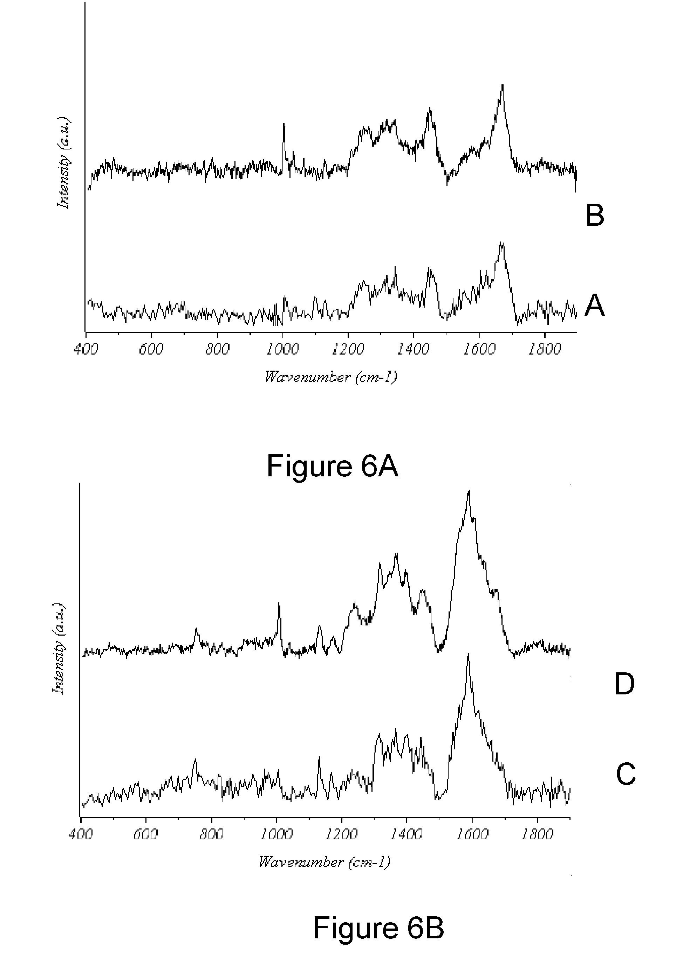

The present design has been optimised so that spectral markers and/or indicators of change to a sample may be identified, and the acquisition procedure can be simplified using low resolution Raman systems. Thus the high resolution and sensitivity of benchtop systems may be sacrificed for the classification of samples which have been well characterised according to spectroscopic markers or indicators, thus allowing for smaller, less expensive systems and faster testing times. In particular, low resolution Raman spectroscopy (LRRS) may be implemented with low cost lasers and miniature spectrometers thus lowering the size, weight and cost of instrumentation by an order of magnitude. Experimental work by the inventors has shown that the techniques presented herein are more than adequate to resolve the spectral differences previously identified in high resolution Raman studies (figure 5 and 6) performed by the inventors.

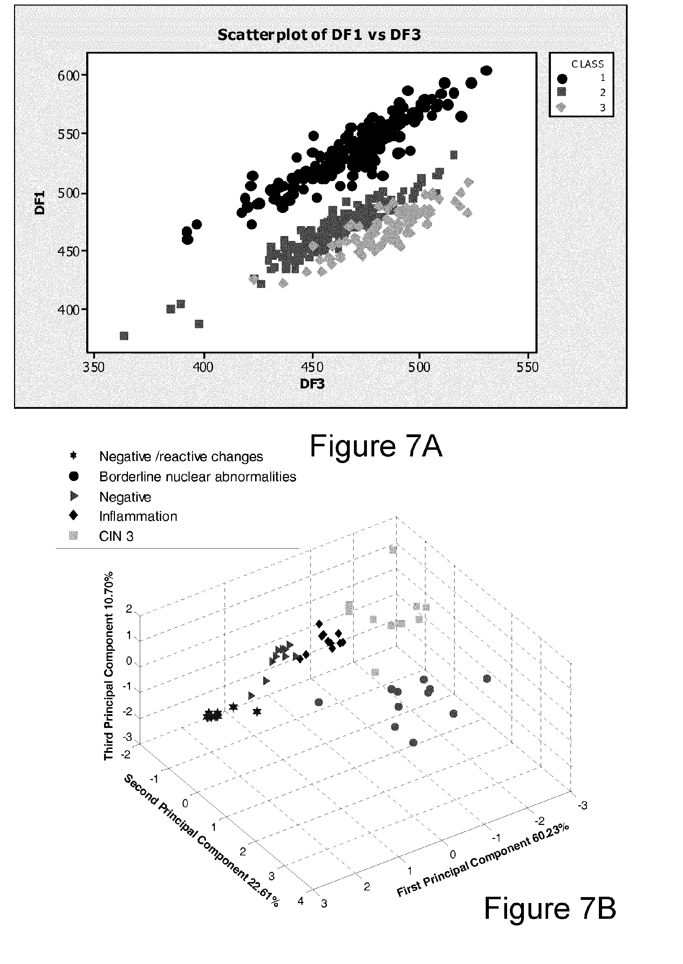

The dimensionality of the data was reduced using principal component analysis (PCA) as described above. The PCA analysis showed good separation between the spectra of normal and abnormal cell and tissue types (figure 7).

For the low resolution spectroscopy of the tissue sections, PCA-LDA was used to classify unknown sections using the previously recorded spectra as a calibration set. A prediction accuracy of **93.4%** was shown. Sensitivity and specificity values of 99.5% and 100% for normal tissue, 94.2% and 92.8% for tumour tissue and 78.9% and 97% for CIN tissue were found. For the low resolution spectroscopy of the smear samples, a support vector machine (SVM) model was trained to allow automatic diagnosis of cells from smear samples. Following rigorous model design and evaluation, the classification model was found to be 100% accurate on unseen data with 100% specificity and 100% sensitivity for normal and abnormal cell types.

Some of the present results were obtained using LDA methods and are indeed encouraging, but alternative analysis methods, which would be familiar to those skilled in the art, including support vector machines (SVM) may prove more beneficial as they have been shown to outperform LDA in a range of applications.

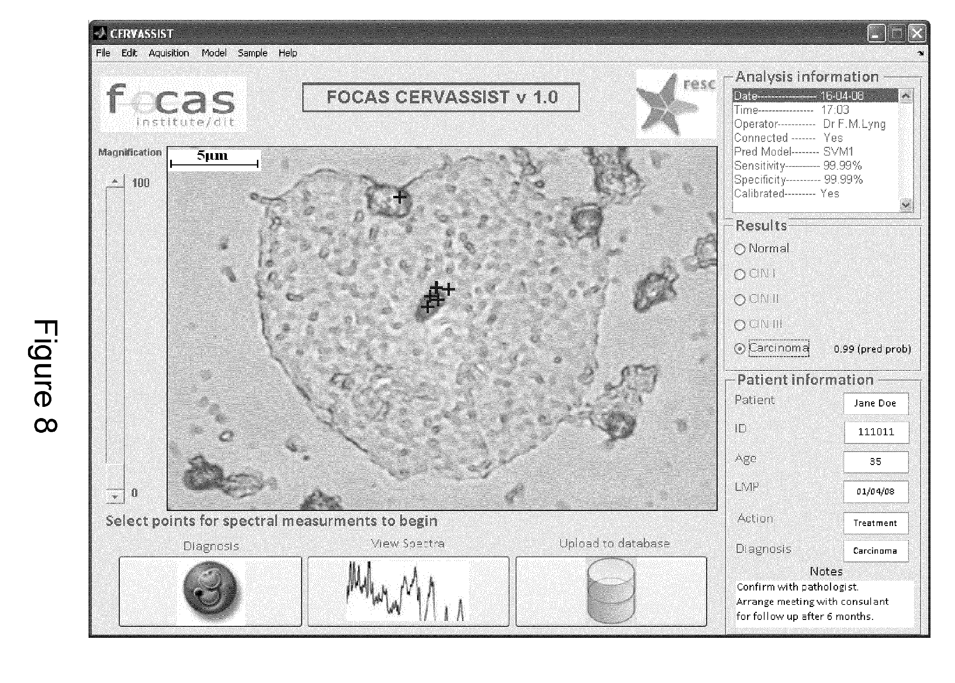

In order to allow non specialist users to operate the classification algorithm, a user friendly graphical user interface (GUI) has been developed as described above and shown by example in Figure 8. The interface allows a user to select an area for acquisition of spectra by the Raman microscope and presents the classification of a selected area. As with other diagnostic systems, the GUI may be connected to a secure relational database for model and diagnosis results. As with other database systems, the clinician may also be able to add patient data and specific sample notes and recommendations for further actions to be taken.

The competitive advantage of the technology is that the low resolution analyser is a compact low cost system that can provide high speed, non subjective diagnosis of cervical cancer and pre-cancer. The output of the system may be a simple colour coded scale graded according to normal, CIN I, II or III, malignant. Advantageously, the system is user friendly for the non specialist user and will give higher specificity and sensitivity values than the currently used cytological methods. Whilst the method\system has been described with reference to pap smears, it will be appreciated that it may be extended to similar tests including investigations of biopsies.

In a further embodiment, a method and system is provided which can provide for an analysis of a sample without necessarily having to identify particular cells on a slide and which may be used as a screening process. The method is particularly suited to the low cost-low resolution arrangement described above.

This further embodiment is best explained by reference to the current methods employed in cytology, whereby cell samples are collected by either exfoliation (scraping from the surface) or fine needle aspiration (sucked up from suspect masses/legions) using a syringe. The cells (held in a liquid preserve) are then either smeared or stamped onto a glass slide for microscopic analysis which involves (subjective) visual analysis of up to hundreds of individual cells in the sample by a cytologist. Whilst the system and methods described above provide for the analysis of these types of sample using Raman spectroscopy, the present inventors, unexpectedly, have discovered that Raman spectroscopy may be performed on a significant mass and not just an individual cell or small group of cells, for example, as presented on a slide in a pap smear.

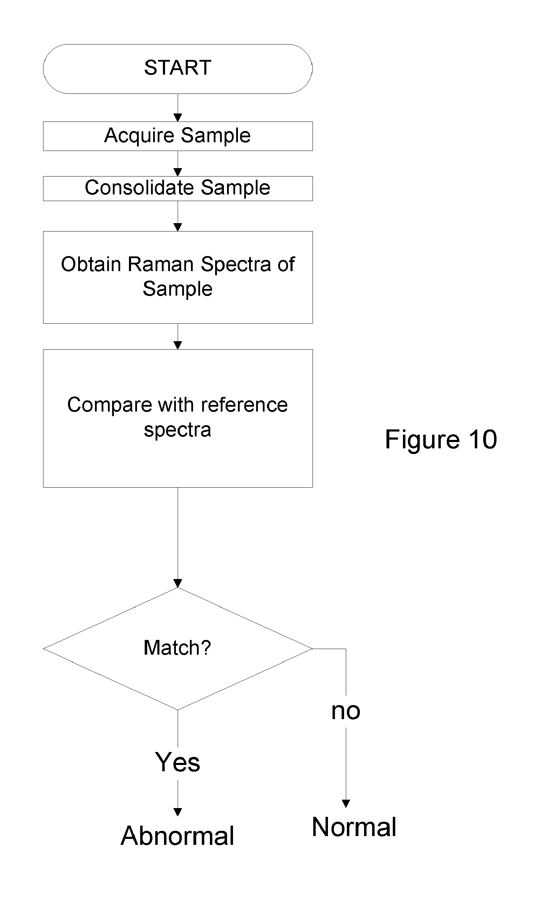

In particular, the present method, rather than spreading out a sample (as performed in a smear), as shown in Figure 10, consolidates the sample into a solid mass. Suitably, this consolidation is performed by a centrifuge. Suitably, the biological sample (cell suspension) to be tested is placed within a centrifuge. The centrifuge is operated at a suitable speed to consolidate the solid matter of the cell suspension into a solid mass (a pellet). Once this has been achieved, the supernatant (the liquid the cells were suspended in) may be discarded. A suitable speed for the centrifuge would be in the range 1000-2000RPM. The centrifuge is suitably operated for a sufficient duration to form the pellet, suitably between 5 and 15 minutes. It will be appreciated that the precise speed and duration of centrifuging may be determined experimentally. In the case of a collected pap smear sample, a suitable speed of 1200 for a duration of 8 minutes has proven reliable during experiments by the inventors.

Once the centrifuging process has been completed, the pellet may be placed on a sample holder, for example a slide. The sample holder may then be placed in the system described above and Raman spectroscopy performed. In contrast to the method described above, the alignment is relatively simple, since there is no need to identify the location of a particular cell on the slide, instead it is sufficient to align the pellet generally. The techniques described above may then be employed to obtain a representative spectrum for the pellet (rather than an individual cell). The representative spectra may then be compared with a library of reference spectra of abnormal cells as described previously. Experimental results have shown that abnormal cells may be identified with a reasonable ratio of abnormal to normal cells. To date, using the analysis techniques described above, abnormal cells have been identified in samples where approximately 30% of the cells were abnormal. It is expected that with further refinement this figure will improve significantly.

The main advantage of this latter method\system is that good representative spectra may be obtained from the entire sample by using the cell pellet rather than having to look at individual cells. Thus, the need for skilled operators is negated since the problems associated with identifying cells and alignment are eliminated. Moreover, the technique opens up the possibility of using a simpler RAMAN spectroscopy system where the need for a microscope is obviated since the pellet is visible and thus may be aligned by direct observation. Indeed, the alignment step may be inherent depending on the holder employed for the pellet. Additionally, the possibilities of employing fibre optic Raman spectroscopy becomes available, whereby the end of the fibre probe is merely directed at the pellet. This provides for a higher throughput analysis of cytological samples. The samples could be screened quickly and easily using an inexpensive portable Raman spectrometer with a fibre optic probe.

An advantage of the present system is that it may be applied to a variety of different applications in cytology, with different reference libraries employed depending on the cytology application, e.g. Gynaecological, Breast, Urinary, Effusion, Thyroid and Lymph Node.

It will be appreciated that a variety of modifications may be made to the systems and methods described herein without departing from the scope of the present application.

## Claims

1. A cytology system for analyzing a biological sample on a sample holder, optionally a slide, the system comprising: a stage for receiving the sample holder, a low resolution Raman spectroscopy device having a spectral resolution worse than 3 wavenumbers, the Raman spectroscopy device having an analysis module for determining whether the spectrum falls within one or more predefined classes of cell.

2. A system according to claim 1, wherein the biological sample is a Pap smear on a glass slide.

3. A system according to claim 1 or claim 2, wherein the one or more predefined classes comprise the following: a) normal b) invasive carcinoma and c) cervical intraepithelial neoplasia (CIN).

4. A system according to claim 3, wherein the CIN class is further delineated into the classes of: a) CIN I b) CIN II and C) CIN III.

5. A system according to any preceding claim, wherein the image analysis identifies cells as areas of interest.

6. A system according to any preceding claim, further comprising: a controller, and a microscope for viewing the sample holder, the microscope having a central optical axis, wherein the Raman spectroscopy device shares the central optical axis of the microscope, and wherein the controller is adapted to cause the stage to move an identified area of interest on the slide to be in-line with the central optical axis and to cause a spectrum to be obtained by the Raman spectroscopy device for the area of interest,

7. A system according to claim 6, further comprising a graphical user interface comprising a window display the view from the microscope, wherein the interface is configured to allow a user to use a pointer to identify the area of interest.

8. A system according to claim 7 wherein the result of the determination of whether the spectrum falls within one or more predefined classes of cell is displayed within the graphical user interface.

9. A system according to any one of claims 6 to 8, wherein the analysis module is configured to perform image analysis on an image acquired by the microscope to identify areas of interest.

10. A system according to any one of claims 6 to 9, wherein the system further comprises a light source for illuminating the slide, wherein the controller is adapted to switch off the light source when operating the Raman spectroscopy device.

11. A system according to any one of claims 6 to 10 further comprising a moveable mirror for switching the optical path between the microscope and the Raman spectroscopy device, wherein the moveable mirror is responsive to the controller.

12. A method of analyzing biological samples where the biological sample is a Pap smear, the method comprising the steps of:
performing low resolution Raman sprectroscopy with a spectral resolution worse than 3 wavenumbers to obtain a spectrum for the biological sample, and analysing the spectrum to determine whether the spectrum falls within one or more predefined classes of cells.

13. A method according to claim 12, wherein the one or more predefined classes comprise the following: a) normal b) invasive carcinoma and c) cervical intraepithelial neoplasia (CIN).

14. A method according to claim 13, wherein the CIN class is further delineated into the classes of: a) CIN I b) CIN II and C) CIN III.

15. A method according to any one of claims 6 to 9, further comprising the step of using a centrifuge to consolidate the contents of the biological sample into a pellet prior to performing the Raman spectroscopy, suitably wherein the centrifuge is operated at a speed of between 1000 and 1500 RPM and ideally, approximately 1200 RPM.
